# EUROPÄISCHE PATENTSCHRIFT

(11) **EP 1 566 441 B1**
(45) Veröffentlichungstag und Bekanntmachung des Hinweises auf die Patenterteilung: **18.02.2009**
(21) Anmeldenummer: 05001539.5
(22) Anmeldetag: 18.12.2001
(51) Int. Cl.: C12N 15/60, C12N 9/88, C12P 13/00

(54) **Gene, enthaltend eine für Hydroxynitrillyase codierende DNA-Sequenz, rekombinante Proteine mit Hydroxynitrillyase-Aktivität und deren Verwendung**
Genes coding for hydroxynitrile lyase, recombinant proteins with hydroxynitrile lyase activity and their use
Gènes codant pour des hydroxynitrile lyases, protéines récombinantes à activité d'hydroxynitrile lyase et leur utilisation

(30) Priorität: 16.01.2001 AT 602001; 03.04.2001 AT 5232001
(43) Veröffentlichungstag der Anmeldung: 24.08.2005
(62) Teilanmeldung aus: 01130058.9
(73) Patentinhaber: DSM Fine Chemicals Austria Nfg GmbH & Co KG, 4021 Linz (AT)
(72) Erfinder: Schwab, Helmut, 8043 Graz (AT); Glieder, Anton, 8200 Gleisdorf (AT); Kratky, Christoph, 8043 Graz (AT); Dreveny, Ingrid, 8010 Graz (AT); Pöchlauer, Peter, 4040 Linz (AT); Skranc, Wolfgang, 1220 Wien (AT); Mayrhofer, Herbert, 4210 Engerwitzdorf (AT); Wirth, Irma, 4470 Enns (AT); Neuhofer, Rudolf, 4210 Mittertreffling (AT); Bona, Rudolfo, 8055 Graz (AT)
(74) Vertreter: Habets, Winand

(56) Entgegenhaltungen:
- EP-A- 0 276 375
- WO-A-97/03204
- HU ZIHUA ET AL: "Molecular analysis of (R)-(+)-mandelonitrile lyase microheterogeneity in black cherry." PLANT PHYSIOLOGY (ROCKVILLE), Bd. 119, Nr. 4, April 1999 (1999-04), Seiten 1535-1546, XP002195510 ISSN: 0032-0889 -& DATABASE EMBL 1. Oktober 1998 (1998-10-01), "Prunus serotina (R)-(+)-mandelonitrile lyase isoform MDL5 precursor (MDL5), mRNA, complete cds." XP002195848 Database accession no. AF053886 -& DATABASE EMBL 16. Dezember 1996 (1996-12-16), "Prunus serotina (R)-(+)-mandelonitrile lyase isoform MDL1 precursor gene, complete cds." XP002195849 Database accession no. U78814
- SUELVES MONICA ET AL: "Molecular cloning of the cDNA coding for the (R)-(+)-mandelonitrile lyase of Prunus amygdalus: Temporal and spatial expression patterns in flowers and mature seeds." PLANTA (BERLIN), Bd. 206, Nr. 3, 11. Oktober 1998 (1998-10-11), Seiten 388-393, XP002195511 ISSN: 0032-0935 -& DATABASE EMBL 24. September 1996 (1996-09-24), "P. amygdalus mRNA for mandelonitrile lyase" XP002195850 Database accession no. Y08211
- EFFENBERGER F: "ENZYME-CATALYZED PREPARATION AND SYNTHETIC APPLICATIONS OF OPTICALLY ACTIVE CYANOHYDRINS" CHIMIA, AARAU, CH, Bd. 53, Nr. 12, 1999, Seiten 3-10, XP000986127 ISSN: 0009-4293
- CEREGHINO JOAN LIN ET AL: "Heterologous protein expression in the methylotrophic yeast Pichia pastoris." FEMS MICROBIOLOGY REVIEWS, Bd. 24, Nr. 1, Januar 2000 (2000-01), Seiten 45-66, XP002195512 ISSN: 0168-6445

## Beschreibung

Biokatalytische Verfahren haben für die chemischen Industrie eine hohe Bedeutung erlangt. Die Durchführung chemischer Reaktionen unter Zuhilfenahme biologischer Katalysatoren ist dabei vor allem in solchen Anwendungsgebieten von Interesse, in denen die oft vorhandene Eigenschaft von Enzymen, bei chemischen Reaktionen mit chiralen oder prochiralen Komponenten eines der beiden Enantiomeren bevorzugt umzusetzen oder zu bilden, genutzt werden kann.

Wesentliche Voraussetzungen für die Nutzung dieser günstigen Eigenschaften von Enzymen sind ihre Verfügbarkeit in technisch benötigten Mengen und eine ausreichend hohe Reaktivität, sowie Stabilität unter den realen Bedingungen eines technischen Verfahrens.

Eine besonders interessante Klasse von chiralen chemischen Verbindungen sind Cyanhydrine. Cyanhydrine sind beispielsweise bei der Synthese von α-Hydroxysäuren, α-Hydroxyketonen, β-Aminoalkoholen, die zur Gewinnung biologisch wirksamer Stoffe, z.B. pharmazeutischer Wirkstoffe, Vitamine oder pyrethroider Verbindungen Verwendung finden, von Bedeutung.

Die Herstellung dieser Cyanhydrine erfolgt durch Addition von Blausäure an die Carbonylgruppe eines Ketons oder Aldehyds.

Die technische Herstellung von chiralen Verbindungen, wie etwa (S)-Cyanhydrinen, konnte durch die Erschließung des Enzyms (S)-Hydroxynitril Lyase aus *Hevea brasiliensis* realisiert werden und ist beispielsweise in WO 97/03204, EP 0 951561 und EP 0 927 766 beschrieben.

Es gibt jedoch eine Vielfalt an interessanten chemischen Verbindungen, bei welchen die R-Enantiomeren für technische Anwendungen von Bedeutung sind. Bisher wurden lediglich Verfahren zur Herstellung einer Reihe von Produkten beschrieben, die nur im Labormaßstab eingesetzt werden können (z.B.: EP 0 276 375, EP 0 326 063, EP 0 547 655). Dabei wurden vorwiegend Enzympräparationen eingesetzt, die aus Pflanzen der Familie Rosaceae, beispielsweise aus den Kernen von Mandeln *(Prunus amygdalus)* gewonnen wurden.

In letzter Zeit gewannen *Prunus spezies* immer mehr an Bedeutung, sodass versucht wurde, diese genauer zu erforschen.

Aus der Fachliteratur, beispielsweise aus Plant Physiology, April 1999, Vol 119, pp. 1535-1546, ist bekannt, dass in *Prunus spezies* mehrere R-HNL Isoenzyme vorkommen können. Diese Isoenzyme sind in verschiedenen Geweben der Pflanze unterschiedlich stark exprimiert. Bei der zu *Prunus amygdalus* nahe verwandten Pflanze *Prunus serotina* konnten bislang 5 verschiedene Isoenzyme identifiziert und deren Gene sequenziert werden. Von *Prunus amygdalus* ist bislang nur ein Isoenzym in Planta (1998) 206: 388-393 beschrieben worden, nämlich eines das in der Blütenknospe am stärksten exprimiert ist. Ein Gen für dieses R-HNL Isoenzyme wurde bereits isoliert und die cDNA sequenziert.

Es ist jedoch noch kein Bericht einer erfolgreichen (funktionellen) heterologen Expression eines solchen Gens in der Fachliteratur oder Patentliteratur beschrieben.

Auch technische Anwendungen im größeren Maßstab sind bislang nicht realisiert worden. Die wesentliche Ursache dafür ist, dass Enzympräparationen aus Mandelkernen mit Hydroxynitrillyase-Aktivität bisher nicht in ausreichenden Mengen und zu vertretbaren Kosten verfügbar waren.

Ziel dieser Erfindung war es daher eine Basis zu schaffen, die eine R-Hydroxynitrillyase in für technische Anwendungen benötigten Mengen bereitstellen kann.

Zur Erreichung dieses Zieles wurde ein Weg gesucht, ein der R-HNL Präparation von *Prunus amygdalus* entsprechendes Enzym durch gentechnische Strategien mit Hilfe eines entsprechenden rekombinanten Mikroorganismenstammes zu produzieren. Ein solches rekombinates Enzym mit R-HNL Aktivität sollte auf diese Art in ausreichender Menge technisch verfügbar gemacht werden.

Aus den in obiger Literatur beschriebenen DNA Sequenzen kann abgeleitet werden, dass bei den Genen der verschiedenen Isoenzyme bestimmte Bereiche hoch konserviert sind. Das wird erfindungsgemäß als Grundlage herangezogen, Primer für die PCR-Amplifikation von *P.amygdalus* R-HNL Genen zu generieren. Mit Hilfe solcher Primer können dann mit aus beispielsweise Mandelkernen *(P. amygdalus)* isolierter DNA als Template (Vorlage) mittels PCR DNA-Stücke amplifiziert werden, die bei Analyse durch Agarose-Gelelektrophorese konkrete spezifische Banden zeigen. Erfindungsgemäß waren dabei Banden zu finden, die der Größe von mdl Genen (Planta (1998) 206: 388-393) entsprachen. Anschließend werden entsprechende Primer für alle bei *Prunus serotina* bekannten Isoenzyme generiert und entsprechende PCR Produkte gewonnen. DNA aus diesem Bereich wird aus entsprechenden präparativen Agarosegelen isoliert und in Standardvektoren für die Klonierung von PCR-generierten Fragmenten in *Escherichia coli* einkloniert.

Die Sequenzanalyse von einer Serie ausgewählter Klone ergab, dass Klone mit Homologien zu den jeweiligen bereits bekannten R-HNL Genen von Prunusarten vorhanden sind, obwohl sich die Sequenzen der so erhaltenen Klone bzw. Gene in mehreren Sequenzpositionen von den bereits bekannten bzw. publizierten Sequenzen unterscheiden, wodurch wichtige funktionelle Unterschiede festgelegt werden.

Es wurde daher unerwarteterweise eine neue Variante der HNL Gene gefunden, obwohl Primerkombinationen verwendet wurden, bei der die bereits bekannte Sequenz einer aus Blütenmaterial von *Prunus amygdalus* gewonnenen cDNA herangezogen wurde

Die neuen Gene wurden sequenziert und die genomische DNA-Sequenz-ermittelt.

So können beispielsweise genspezifische PCR Primer basierend auf der Sequenzhomologie des MDL1 Gens von *Prunus amygdalus* und des *mdl5* Gens von *Prunus serotina* hergestellt werden, worauf nach Amplifikation und Klonierung das *HNL5* Gen, erhalten wird.

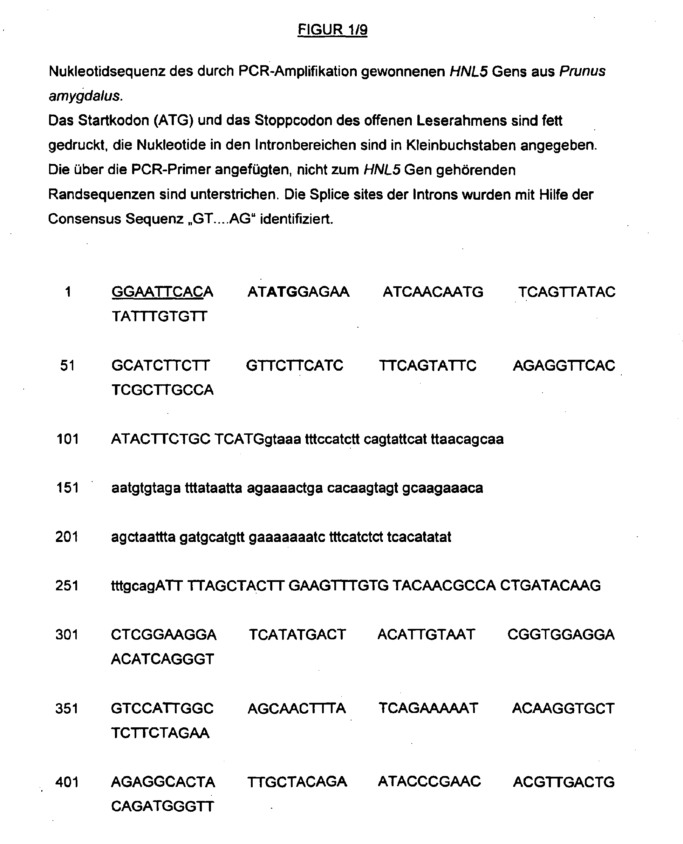

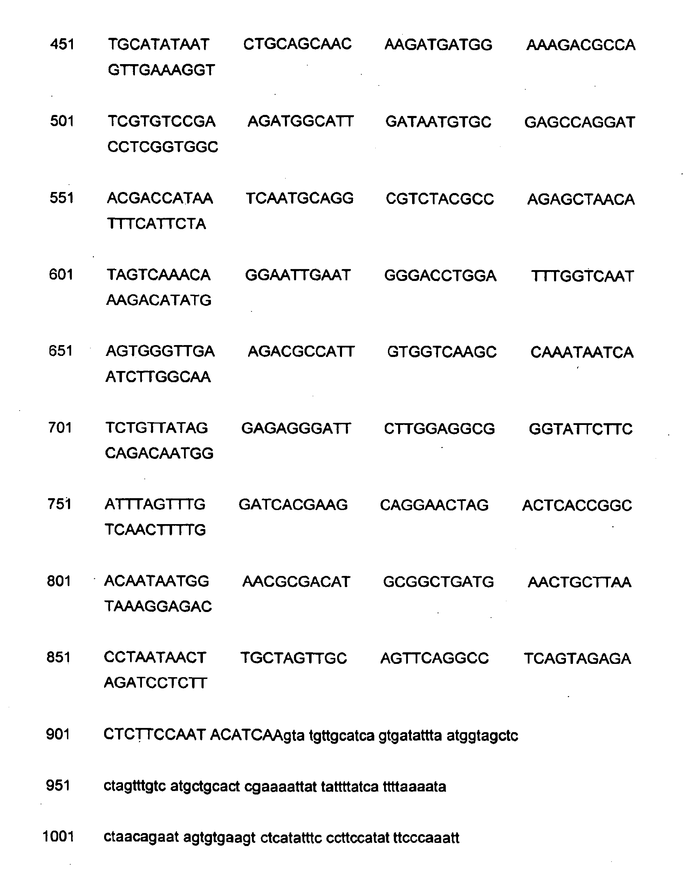

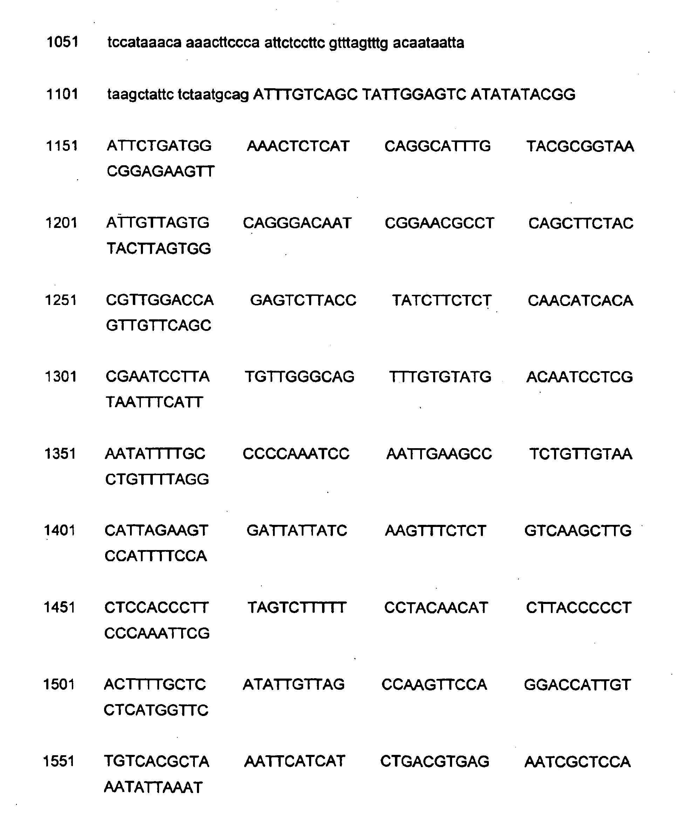

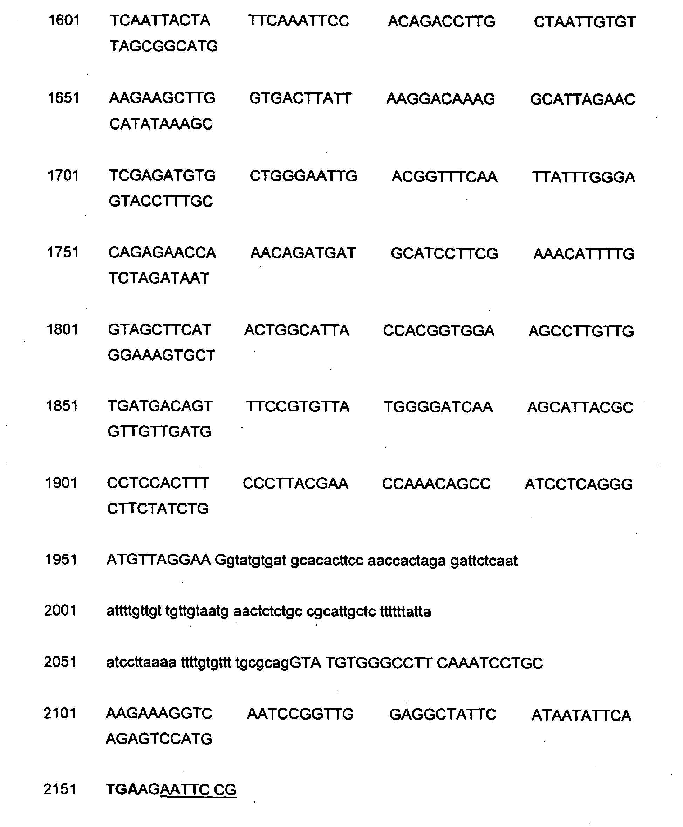

Beispielsweise weist das durch PCR-Amplifikation gewonnene *HNL5* Gen aus *Prunus amygdalus* die in Figur 1 abgebildete Nukleotidsequenz auf.

Weiters können beispielsweise genspezifische PCR Primer basierend auf der Sequenzhomologie des *mdl1* Gens von *Prunus serotina* hergestellt werden, worauf nach Amplifikation und Klonierung ein neues Gen, das *HNL1Gen,* erhalten wird.

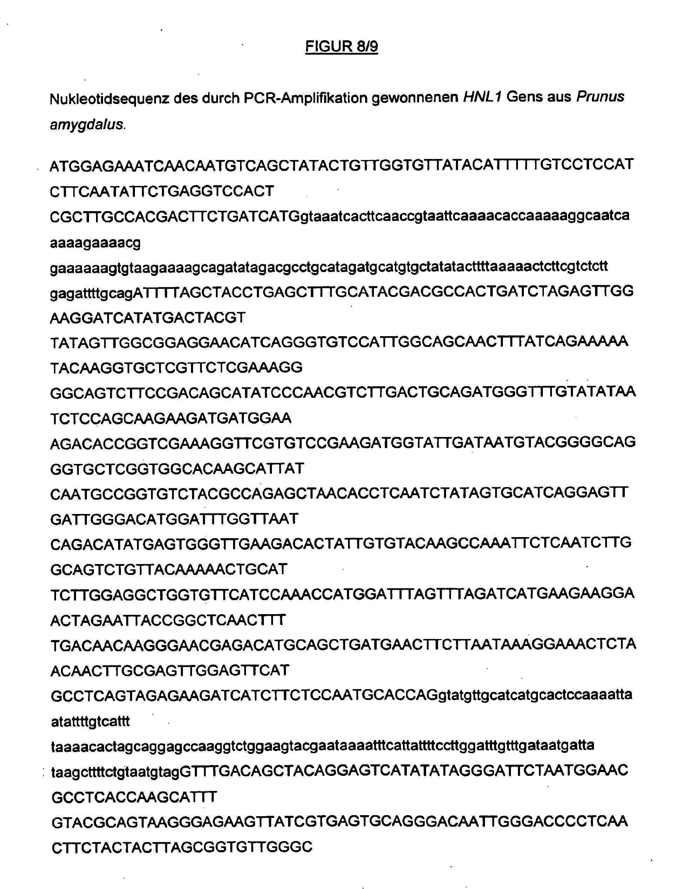

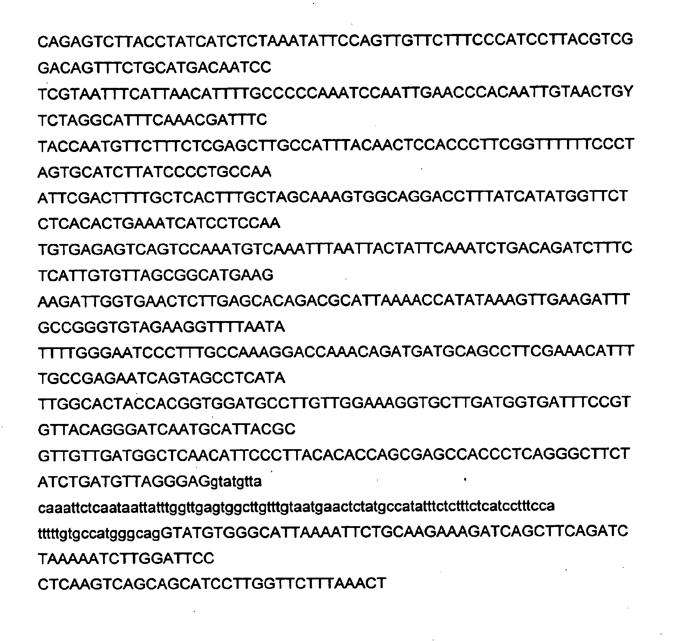

Beispielsweise weist das durch PCR-Amplifikation gewonnene *HNL1* Gen die in Figur 8 abgebildete Nukleotidsequenz auf, die Gegenstand der Erfindung ist.

Gegenstand der Erfindung ist eine Isolierte Nukleinsäure, welche die in Figur 8 abgebildete Nukleotidsequenz von Nukleotid 1 bis Nukleotid 2083 durchgehend oder diese Sequenz abzüglich der Intron-Bereiche von den Nukleotiden 104 bis 249, 907 bis 1047 und 1889 bis 1993 umfasst.

Die genomischen Klone bzw. deren genomische DNA stellen die Basis für die Gewinnung von Enzympräparationen durch heterologe Expression, beispielsweise durch induzierbare oder konstitutive Expression, in verschiedenen Wirtszellen dar.

Weiters ist aus der Sequenzanalyse der genomischen DNA der neuen, erfindungsgemäßen Gene ersichtlich, dass es sich bei dem von den neuen Genen kodierten Proteinen um Proteine handelt, die über eine Signalsequenz bzw. ein pflanzliches Signalpeptid verfügen, wodurch auch eine sekretorische Expression von heterologen Proteinen in geeigneten Wirtszellen ermöglicht wird.

Dabei werden spezielle Expressionsvektoren verwendet, mit denen das von einem der einklonierten neuen Gene codierte Protein als Fusionsprotein mit einem Signalpeptid exprimiert wird.

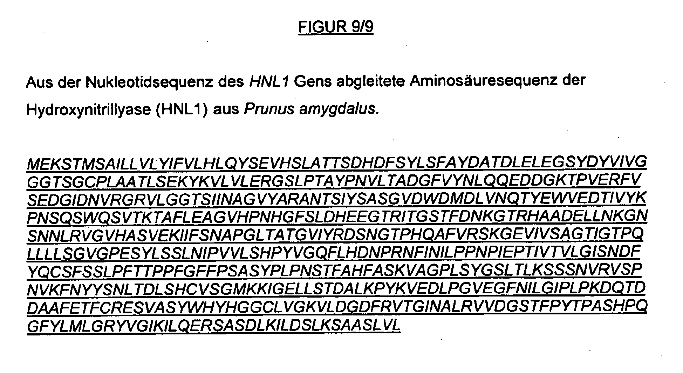

Die vorliegende Erfindung betrifft demnach weiters rekombinante Proteine, herstellbar durch heterologe Expression der DNA-Sequenz der *HNL1* Gene aus *Prunus amygdalus* in geeigneten Wirtszellen, die die in Fig. 9 abgebildete Aminosäuresequenzen aufweisen.

Geeignete Wirtszellen sind dabei beispielsweise Mikroorganismen. Bevorzugt sind eukaryotische Mikroorganismen, besonders bevorzugt Pilze. Beispiele dafür sind *Saccharomyces cerevisiae* oder *Pichia pastoris.*

Beispielsweise ist die aus der Nukleotidsequenz des *HNL1* Gens abgeleitete Aminosäuresequenz der Hydroxynitrillyase HNL1 aus *Prunus amygdalus* in Figur 9 dargestellt.

Geeignete Wirtszellen sind dabei wiederum beispielsweise Mikroorganismen. Bevorzugt sind Bakterien oder eukaryotische Mikroorganismen, besonders bevorzugt Pilze, wie etwa *Saccharomyces cerevisiae* oder *Pichia pastoris.*

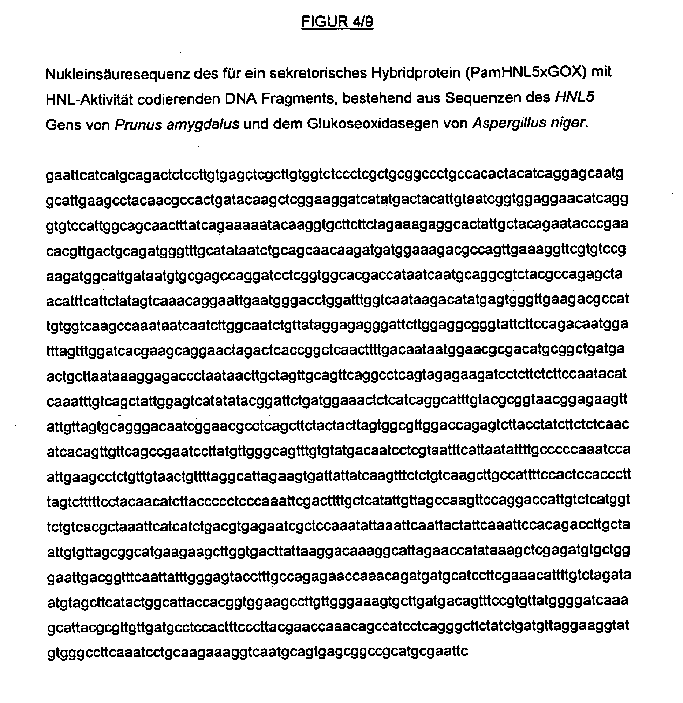

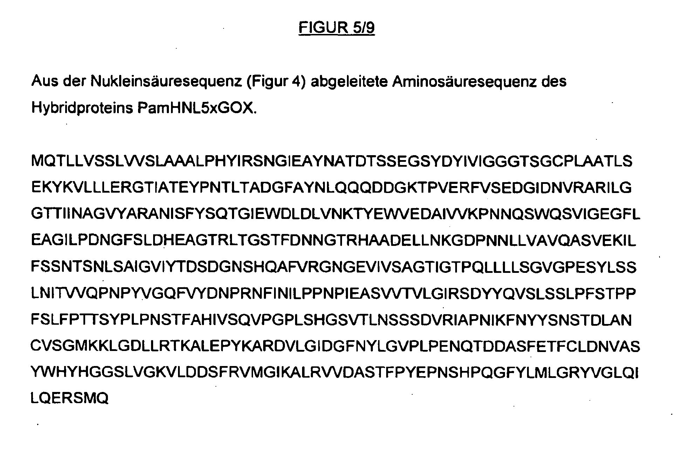

Beispielsweise ist die Nukleinsäuresequenz des für ein sekretorisches Hybridprotein (PamHNL5xGOX) mit HNL-Aktivität codierenden DNA Fragments, bestehend aus Sequenzen des *HNL5* Gens von *P. amygdalus* und dem Glukoseoxidase-Gen von *Aspergillus niger* in Figur 4 abgebildet. Die aus der Nukleotidsequenz (Figur 4) abgeleitete Aminosäuresequenz des Hybridproteins PamHNL5xGOX ist in Figur 5 dargestellt.

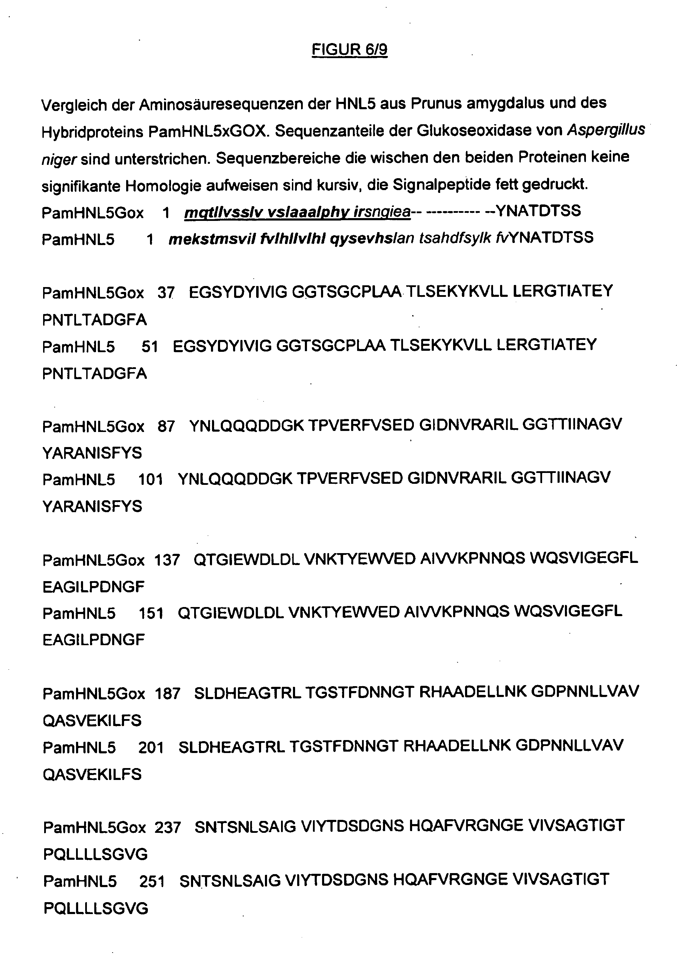

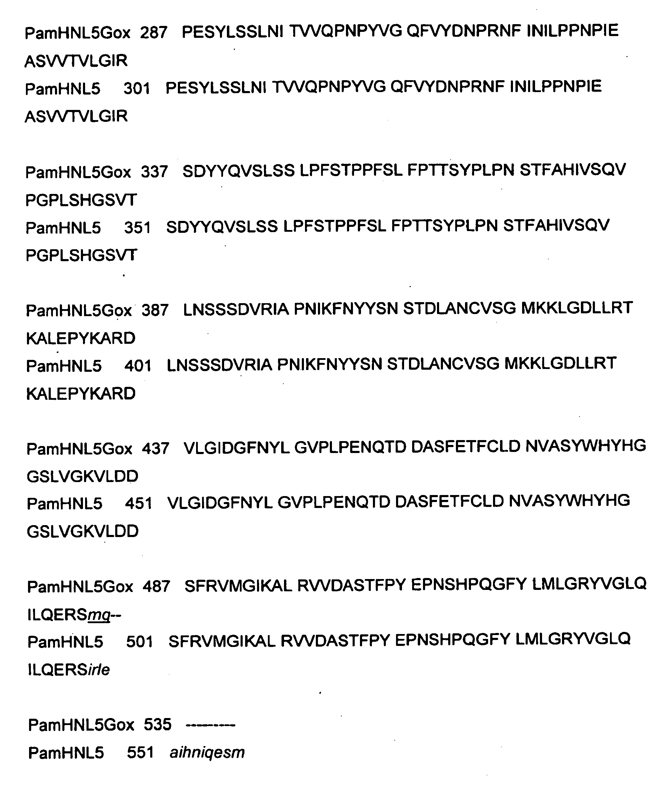

Figur 6 zeigt den Vergleich der Aminosäuresequenzen de HNL5 Proteins aus *Prunus amygdalus* und des Hybridproteins PamHNL5xGOX.

Um zu den erfindungsgemäßen rekombinanten Proteinen zu gelangen, werden beispielsweise die Klone, die Homologien zu den bekannten Genen von MDL1 von *P. amygdalus* und/oder für mdl1 von *P.serotina* und zeigen, weiter bearbeitet.

Um rekombinante Proteine mit Hydroxynitrillyase Aktivität zu erhalten werden die entsprechenden *HNL1* Gene z.B. in einen Expressionsvektor für *Pichia pastoris* oder für *Saccharomyces cerevisiae* eingebaut.

Zuvor kann die genomische DNA mittels PCR gespleisst werden. Bevorzugt wird ein Basisfragment zur Konstruktion von Expressionsplasmiden für die heterologe Expression des entsprechenden Gens in Bakterien und Eukaryonten hergestellt. Dazu wird ein Plasmid konstruiert, aus dem ein für ein erfindungsgemäßes Gen kodierendes DNA Fragment für den Einbau in verschiedene Expressionsvektoren durch Ausschneiden mit Restriktionsendonukleasen gewonnen werden kann.

Mit den erfindungsgemäßen Genen kann somit durch Expression, beispielsweise mit einem induzierbaren Promotersystem oder einem konstitutiv exprimierenden Promotorsystem, in einem heterologen Wirt eine funktionelle HNL produziert werden.

Aus einer großen Anzahl von Transformanten können somit rekombinante Stämme von beispielsweise *Pichia pastoris* gefunden werden, die rekombinantes Protein mit R-HNL Aktivität überexprimieren. Das Protein mit R-HNL Aktivität ist bei diesen Stämmen nach Induktion der Expression zum überwiegenden Teil im Kulturüberstand zu finden.

Somit konnte erstmals ein rekombinantes Protein in für technische Anwendungen nutzbaren Mengen hergestellt werden, das eine zu in Mandelkernen (Kerne von *Prunus amygdalus)* aufzufindende vergleichbare R-HNL Aktivität aufweist.

Unerwarteterweise konnte festgestellt werden, dass die rekombinanten Proteine mit R-HNL Aktivität, die sich beispielsweise von den erfindungsgemäßen *HNL1* Genen ableiten und mit Pam-HNL1 bezeichnet werden, im Vergleich zum aus Mandelkernen isolierten Enzympräparat wesentlich bessere Eigenschaften als Biokatalysator in Reaktionsansätzen zur Herstellung von Cyanhydrinen aufweisen und somit besonders vorteilhaft für die biokatalytische Synthese von Cyanhydrinen verwendet werden können.

Die Sequenzen der erfindungsgemäßen rekombinanten Proteine können weiters auch noch am C-terminalen Ende verkürzt werden, bzw. können die Sequenzen im N- und C-terminalen Bereich durch solche eines verwandten Proteins mit anderer Funktion ausgetauscht werden. Derart abgewandelte Proteine sind demnach auch ein Gegenstand der Erfindung.

Die rekombinanten Proteine zeichnen sich insbesondere auch dadurch aus, dass sie eine wirtsspezifische Glykosilierung aufweisen, wodurch die rekombinanten Proteine wesentlich stabiler sind als die nativen Proteine.

Ein besonderer Vorteil der erfindungsgemäßen rekombinanten Proteine ergibt sich durch die wesentlich höhere Stabilität, durch die wesentlich weniger Enzymmenge im Vergleich zu nativem Enzym benötigt wird um hohe Enantiomerenreinheit zu erzeilen. So zeigten Vergleichsversuche, dass bei Verwendung der erfindungsgemäßen rekombinanten Proteine lediglich ein Zehntel der benötigten Menge an nativem Protein benötigt wird, um vergleichbare Enantiomerenreinheiten (ee-Werte) zu erzielen.

Ein weiterer Gegenstand der Erfindung ist demnach die Verwendung der erfindungsgemäßen rekombinanten Proteine (HNLs) zur Herstellung von (S)- oder (R)-Cyanhydrinen.

Als Ausgangsmaterialien zur Herstellung der (S)- oder (R)-Cyanhydrinen werden ein Aldehyd oder ein Keton als Substrat, ein Cyanidgruppendonor und ein erfindungsgemäßes rekombinantes Protein eingesetzt.

Unter Aldehyden sind dabei aliphatische, aromatische oder heteroaromatische Aldehyde zu verstehen. Unter aliphatischen Aldehyden sind dabei gesättigte oder ungesättigte, aliphatische, geradkettige, verzweigte oder cyclische Aldehyde zu verstehen. Bevorzugte aliphatische Aldehyde sind geradkettige Aldehyde mit insbesondere 2 bis 30 C-Atomen, bevorzugt von 2 bis 18 C-Atomen, die gesättigt oder ein- oder mehrfach ungesättigt sind. Der Aldehyd kann dabei sowohl C-C-Doppelbindungen als auch C-C-Dreifachbindungen aufweisen. Die aliphatischen, aromatischen oder heteroaromatischen Aldehyde können weiters unsubstituiert oder durch unter den Reaktionsbedingungen inerte Gruppen, beispielsweise durch gegebenenfalls substituierte Aryl- oder Heteroarylgruppen, wie Phenyl-, Phenoxy- oder Indolylgruppen, durch Halogen-, Hydroxy-, Hydroxy-C₁-C₅-Alkyl, C₁-C₅-Alkoxy-, C₁-C₅-Alkylthio-, Ether-, Alkohol-, Carbonsäureester-, Nitro- oder Azidogruppen substituiert sein.

Beispiele für aromatische oder heteroaromatische Aldehyde sind Benzaldezyd bzw. verschieden substituierte Benzaldehyde wie etwa 3,4-Difluorbenzaldehyd, 3-Phenoxybenzaldehyd, 4-Fluor-3-phenoxybenzaldehyd, Hydroxybenzaldehyd, Methoxybenzaldehyd, weiters Furfural, Methylfurfural, Anthracen-9-carbaldehyd, Furan-3-carbaldehyd, Indol-3-carbaldehyd, Naphthalin-1-carbaldehyd, Phthaldialdehyd, Pyrazol-3-carbaldehyd, Pyrrol-2-carbaldehyd, Thiophen-2-carbaldehyd, Isophthalaldehyd oder Pyridinaldehyde, Thienylaldehyde u.s.w.

Ketone sind aliphatische, aromatische oder heteroaromatische Ketone, bei denen das Carbonylkohlenstoffatom ungleich substituiert ist. Unter aliphatischen Ketonen sind gesättigte oder ungesättigte, geradkettige, verzweigte oder cyclische Ketone zu verstehen. Die Ketone können gesättigt oder ein- oder mehrfach ungesättigt sein. Sie können unsubstituiert, oder durch unter Reaktionsbedingungen inerte Gruppen, beispielsweise durch gegebenenfalls substituierte Aryl- oder Heteroarylgruppen wie Phenyl- oder Inolylgruppen, durch Halogen-, Ether-, Alkohol-, Carbonsäureester-, Nitro- oder Azidogruppen substituiert sein.

Beispiele für aromatische oder heteroaromatische Ketone sind Acetophenon, Indolylaceton u.s.w.

Aldehyde und Ketone, die sich erfindungsgemäß eignen, sind bekannt oder wie üblich herstellbar.

Die Substrate werden in Anwesenheit der erfindungsgemäßen HNLs mit einem Cyanidgruppendonor umgesetzt.

Als Cyanidgruppendonor kommen Blausäure, Alkali-Cyanide oder ein Cyanhydrin der allgemeinen Formel I

R₁R₂C(OH)(CN),

in Betracht. In der Formel I bedeuten R₁ und R₂ unabhängig voneinander Wasserstoff oder eine unsubstituierte Kohlenwasserstoffgruppe, oder R₁ und R₂ gemeinsam eine Alkylengruppe mit 4 oder 5 C-Atomen, wobei R₁ und R₂ nicht gleichzeitig Wasserstoff bedeuten. Die Kohlenwasserstoffgruppen sind aliphatische oder aromatische, bevorzugt aliphatische Gruppen. Bevorzugt bedeuten R₁ und R₂ Alkylgruppen mit 1 - 6 C-Atomen, ganz bevorzugt ist der Cyanidgruppendonor Acetoncyanhydrin.

Die Herstellung des Cyanidgruppendonors kann nach bekannten Verfahren erfolgen. Cyanhydrine, insbesonders Acetoncyanhydrin, sind auch käuflich zu erwerben.

Bevorzugt wird Blausäure (HCN), KCN, NaCN, oder Acetoncyanhydrin, besonders bevorzugt Blausäure als Cyanidgruppendonor eingesetzt.

Die Blausäure kann dabei auch erst kurz vor der Reaktion aus einem ihrer Salze wie etwa NaCN oder KCN freigesetzt und in Substanz oder in gelöster Form dem Reaktionsgemisch zugegeben werden.

Die Umsetzung kann im organischen, wäßrigen oder 2-Phasensystem oder in Emulsion durchgeführt werden.

Als wäßriges System wird eine wäßrige, die erfindungsgemäße HNL enthaltende Lösung oder Pufferlösung verwendet. Beispiele dafür sind Na-Citratpuffer, Phosphatpuffer u.s.w.

Als organisches Verdünnungsmittel können mit Wasser nicht oder geringfügig mischbare aliphatische oder aromatische Kohlenwasserstoffe, die gegebenenfalls halogeniert sind, Alkohole, Ether oder Ester oder Gemische davon verwendet werden. Bevorzugt werden Methyl -tert.Butylether (MTBE), Diisopropylether, Dibutylether und Ethylacetat oder deren Gemisch eingesetzt.

Die erfindungsgemäßen HNLs können dabei entweder als solche oder immobilisiert in dem organischen Verdünnungsmittel vorliegen, die Umsetzung kann jedoch auch in einem Zweiphasensystem oder in Emulsion mit nichtimmobilisierter HNL erfolgen.

### BEISPIEL 1:

### Isolierung von genomischer DNA aus Mandelkernen (Kerne von Prunus amygdalus)

Getrocknete Mandelkerne (Farmgold, Chargennummer L4532, Ernte 1999) wurden mit einem Messer fein gehackt und in einer Reibschale mit flüssigem Stickstoff eingefroren und unter flüssigem Stickstoff mit einem Pistill zu einem feinen Pulver aufgerieben. 0,1 Gramm gefrorenes Mandelkernpulver wurden direkt mit 65 °C warmem "Breaking Puffer" (100 mM NaAc; 50 mM EDTA; 500 mM NaCl, eingestellt auf pH 5,5; 1,4% SDS und 20µg / ml RNAse A) versetzt. Nach 15 minütiger Inkubation unter ständigem Schütteln wurden die unlöslichen Zellrückstände durch Zentrifugation (10 min. bei 7000 g) abgetrennt und der Überstand mit einem gleichen Volumen 10M Ammoniumacetat versetzt und anschließend 10 min auf Eis inkubiert. Nach 15 minütiger Zentrifugation bei 10.000g wurde der Überstand 2 x mit Phenol/Chloroform (1/1, Phenol äquilibriert mit 50 mM Tris, pH 8.0) extrahiert. Nach einer weiteren Extraktion mit doppeltem Volumen Chloroformilsoamylalkohol (24/1) wurde die DNA aus dem Überstand mit einem gleichen Volumen Isopropanol gefällt, abzentrifugiert, das DNA Pellet mit 70% Äthanol gewaschen und luftgetrocknet. Die DNA wurde dann für 20 min bei 68°C in 200 µl Wasser gelöst und durch eine Ethanolfällung (Ausubel et al., 1999) gereinigt. Nach der Zentrifugation wurde das DNA Pellet luftgetrocknet und in 50 µl Wasser gelöst.

### BEISPIEL 2:

Amplifikation und Klonierung eines genomischen DNA Abschnittes von Mandel *(Prunus amygdalus)* DNS mit Homologie zu bekannten *mdl* Genen von Rosaceae.

Da bekannt war, dass in *Prunus spezies* mehrere Isoenzyme von Hydroxyntrillylasen mit hoher Sequenzhomologie zueinander auftreten können (Hu und Poulton, 1999), wurden genspezifische PCR Primer basierend auf der Sequenzhomologie des *mdl5* Gens von *Prunus serotina* und des *MDL1* Gens von *Prunus amygdalus* (Suelves et al., 1998) hergestellt:
Primer 1: 5'- CGGAATTCACAATATGGAGAAATCAACAATGTCAG-3'
Primer 2: 5'- CGGAATTCTTCACATGGACTCTTGAATATTATG-3'

Die Amplifikation erfolgte in einem 50 µl Ansatz mit 1,2 U "Hotstar" *Taq* DNA Polymerase (Qiagen, Hilden, Deutschland), mit 50 ng genomischer Mandel-DNA als "Template", je 200 ng Primer 1 und 2, 5 µl eines dNTP (je 2 mM) Mix, alles in 1x PCR Puffer entsprechend dem Manual des "Hotstar Kit" (Qiagen, Hilden, Deutschland), beginnend mit einem 15 minütigen Denaturierungsschritt bei 95°C, gefolgt von 30 Zyklen (1 min 95°C, 30 sec 64°C, 1 min 72°C) zur Amplifikation und einer abschließenden Inkubation für 5 min bei 72 °C zur Herstellung vollständiger Produkte.

Durch diese PCR wurde ein DNA Fragment in der Grösse von ca. 2,16 kb (festgestellt durch Analyse mittels Agrose-Gelelektrophorese) erhalten. Dieses PCR Produkt wurde mittels "Qiaquick Kit" (Qiagen, Hilden, Deutschland) entsprechend dem beigefügten Manual gereinigt und unter Verwendung des "Dye Deoxy Terminator Cycle Sequencing" Kits (Applied Biosystems Inc., Forster City, CA, USA) nach der Strategie des "Primer walking" ausgehend von den beiden zur PCR eingesetzten Primern sequenziert. Die erhaltene DNA Sequenz des insgesamt 2162 Basenpaare langen PCR Fragments ist in Figur 1 dargestellt.

Ca. 0, 5 µg des gereinigten PCR Produktes wurden mit der Restriktionsendonuklease *Eco*RI geschnitten und in den Plasmidvektor pBSSK(-) (Stratagene Cloning Systems, La Jolla, CA, USA) über die *Eco*RI Schnittstelle einkloniert. Das Insert eines resultierenden rekombinanten Moleküls (das entsprechende Plasmid wurde mit pBSPamHNL5g bezeichnet) wurde nach der oben beschriebenen Methode sequenziert und die Sequenz des klonierten Fragmentes war zu 100 % ident mit der Sequenz des oben beschriebenen mit den beiden Primern (1 & 2) erhaltenen PCR Produktes.

### BEISPIEL 3:

Sequenzanalyse des durch PCR Amplifikation mit den Primern 1 und 2 erhaltenen genomischen *Prunus amygdalus* DNA Fragmentes.

Im Bereich des durch PCR amplifizierten und sequenzierten DNA Abschnitts wurde ein offener Leserahmen gefunden, der durch 3 Introns unterbrochen ist. Diese drei Introns wurden mit mit Hilfe der Intron Consensus Sequenz "GT.....AG" identifiziert. Der Leserahmen beginnt mit einem ATG Codon bei Position +13 und endet mit einem Stop Codon bei Position +2151.

Für den kodierenden Bereich wurden die Fragmente von Position 13 bis 115 (Exon I), Position 258 bis 917 (Exon II), 1121 bis 1961 (Exon III) und 2078 bis 2150 (Exon IV) zusammengefügt. Die zusammengefügte DNA Sequenz kodiert für ein Protein mit 559 Aminosäuren und einem berechneten Molekulargewicht von 61 kDa, bzw. 57, 9 kDa für eine N-terminal prozessierte Form. Die Peptidmassen wurden mit Hilfe des Programmpakets GCG (Genetics Computer Group, Wisconsin, USA) berechnet. Für dieses Protein wurde die Bezeichnung *Pam*HNL5 festgelegt.

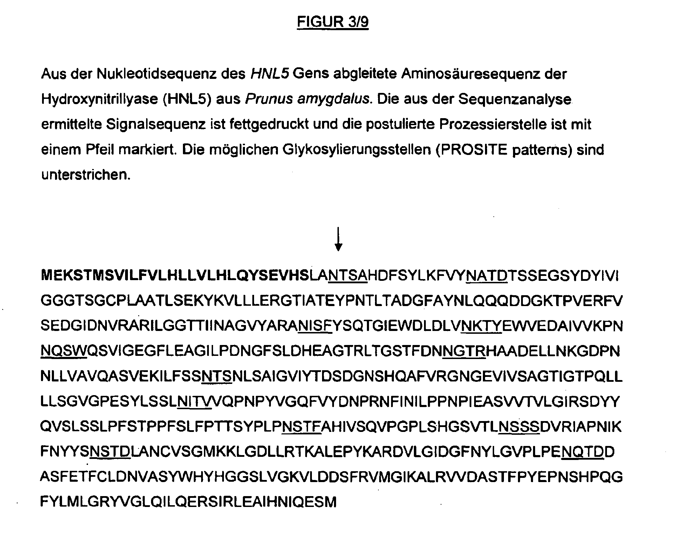

Die für den offenen Leserahmen (ohne Introns) abgeleitete Proteinsequenz ist in Figur 3 gezeigt. Es konnten ausgeprägte Homologien zu bekannten Hydroxynitrillyasen von Rosaceae festgestellt werden ("Blast" Programm, GCG Paket, Version 10.1, Genetics Computer Group, Wisconsin, USA) wobei die höchsten Homologien zu den publizierten Sequenzen von Mdl1 aus *Prunus amygdalus* (99 prozentige Identität, Suelves et al. 1998) und von Mdl5 aus *Prunus serotina* (94 prozentige Identität, Hu und Poulton, 1999) bestehen. Mit Hilfe dieser Homologie wurde auch eine spaltbare Signalsequenz mit Spaltung zwischen S27 und L28 identifiziert. Eine solche Spaltstelle konnte bei den beiden Isoenzymen Mdl1 und Mdl4 von *Prunus serotina* durch N-terminale Sequenzierung der aus Pflanzenmaterial gereinigten nativen Proteine nachgewiesen werden (Zihua Hu und Jonathan E. Poulton, Plant Physiology, 119, 1535 -1546, 1999). Die Sequenzen von verschiedenen in *Rosacea species* vorhandenen HNL Isoenzymen sind nur von *Prunus serotina* bekannt. Aufgrund der höchsten Homologien zur Sequenz von Mdl5 *aus Prunus serotina* wurde das neue *HNL* Gen aus *Prunus amygdalus* als *HNL5* festgelegt. Die Suche nach Sequenzmotiven in der PROSITE-Sequenzmotiv Datenbank (GCG Paket, Version 10.1, Genetics Computer Group, Wisconsin, USA) ergab das Vorhandensein von 13 potentiellen N-Glykosilierungsstellen (in Figur 3 eingezeichnet).

### BEISPIEL 4:

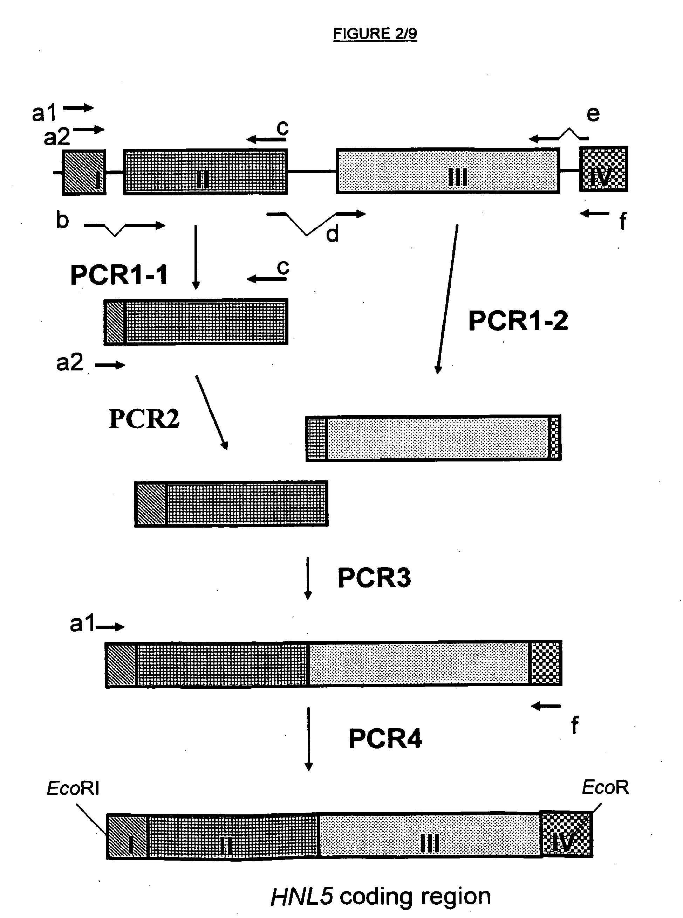

Gewinnung eines intronfreien *Prunus amygdalus HNL5* Gens durch PCR Splicing. Mittels einer speziellen PCR Strategie unter Verwendung von überlappenden Primern wurden die kodierenden Bereiche durch 4 aufeinanderfolgende PCR Reaktionen miteinander verbunden (entsprechend Figur 2).

In der ersten PCR Runde (PCR1-1 und PCR1-2) wurden die Exons II und III mit den Primerpaaren PamHNL5b/PamHNL5c (PCR1-1) bzw. PamHNL5d/PamHNL5e (PCR1-2) amplifiziert. Die 50 µl PCR Ansätze in 1x PCR Puffer (Qiagen) enthielten: je 100 pmol der entsprechenden Primer, 2,5 U "Hotstar" *Taq* DNA Polymerase (Qiagen), 5 µl eines dNTP (je 2 mM) Mix, 10 ng des Plasmids pBSPamHNL5g als Template. Es wurde folgendes Programm gefahren: 15 min 95°C, 30 Zyklen 1 min 95°C, 30 sec. 68°C, 1 min 72 °C und zum Abschluss 5 min 72 °C zur Herstellung vollständiger Produkte). Die Produkte aus PCR1-1 und PCR1-2 wurden nach elektrophoretischer Trennung in einem Agarosegel mittels QiaexII Kit aus dem Gel eluiert.

Durch Amplifikation von ca. 50 ng des Produktes aus PCR1-1 mit je 100 pmol der Primer PamHNL5a2 und PamHNL5c erfolgte in der zweiten PCR-Runde (PCR2) eine Verlängerung dieses ersten PCR Produktes. Es wurde folgendes Programm gefahren: 15 min 95°C, 30 Zyklen 1 min 95 °C, 30 sec. 68°C, 1 min 72°C und zum Abschluss 5 min 72°C zur Herstellung vollständiger Produkte). Die sonstigen Bedingungen waren gleich wie bei PCR1. Dieses PCR Produkt wurde nach elektrophoretischer Trennung ebenfalls über ein Agarosegel gereinigt und eluiert.

Durch primerlose PCR wurden in der dritten PCR-Runde (PCR3) die Produkte aus PCR1-2 und PCR2 mit Hilfe der überlappenden Enden miteinander verbunden (5 Zyklen mit 1 min bei 94°C, 30 sec bei 68°C und 1,5 min 72°C, je ca. 100 ng der beiden Produkte aus PCR1-1 und PCR2 in 50 µl Ansätzen in 1x PCR Puffer (Qiagen), 5 µl des dNTP (je 2 mM) Mix und 2,5 U "Hotstar" *Taq* DNA Polymerase (Qiagen).

Die Ergänzung zur vollen Länge des kodierenden *Prunus amygdalus HNL5* Gens und zur Amplifikation des vollständigen Produktes erfolgte in einer vierten PCR-Runde (PCR4) mit je 100 pmol der Primer PamHNL5a1 und PamHNL5f. Diese wurden direkt zum Reaktionsgemisch der PCR 3 zugegeben. Es wurde folgendes Programm gefahren: 20 Zyklen mit 1 min 95°C, 30 sec. 63°C, 1,5 min 72 °C und zum Abschluss 5 min 72 °C). Die sonstigen Bedingungen waren gleich wie bei PCR1.

Das in der letzten PCR-Runde erhaltene Produkt wurde über ein präparatives Agarosegel aufgetrennt und die DNA mit einer Grösse von 1,6 -1,8 kb mittels "Quiaexll" Kit (Qiagen) aus dem Gel eluiert und über die *E*coRI Schnittstelle in das Plasmid pBSSK(-) einkloniert. Ein Klon mit korrektem Restriktionsmuster wurde ausgewählt und sequenziert.

Die Sequenz im kodierenden Bereich war zu 100 % ident mit den Exons der genomischen DNA Sequenz. Dieser Klon wurde als pBSPamHNL5orf bezeichnet.

### Oligonukleotid-Primer

PamHNL5a1 
5'-
GAAGATCTGAATTCCATGGAGAAATCAACAATGTCAGTTATACTATTTGTGTTGCAT
CTTCTTG-3'
 PamHNL5a2 
5'-
CTATTTGTGTTGCATCTTCTTGTTCTTCATCTTCAGTATTCAGAGGTTCACTCGCTT
GCCAATACTTC-3'
 PamHNL5b 
5'-
GTTCACTCGCTTGCCAATACTTCTGCTCATGATTTTAGCTACTTGAAGTTTGTGTAC
AACGCCACTG-3'
 PamHNL5c
5'-GATGTATTGGAAGAGAAGAGGATCTTCTCTACT-3'
PamHNL5d 
5'
GATCCTCTTCTCTTCCAATACATCAAATTTGTCAGCTATTGGAGTCATATATACGG
3'
 PamHNL5e 
5'-
CAACCGGATTGACCTTTCTTGCAGGATTTGAAGGCCCACATACCTTCCTAACATCA
GATAGAAGCC-3'
 PamHNL5f

5'-
GAAGATCTGGAATTCTTCACATGGACTCTTGAATATTATGAATAGCCTCCAACCGG
ATTGACCTTTCTTGCAG-3'

### BEISPIEL 5

Herstellen eines Basisfragments zur Konstruktion von Expressionsplasmiden für heterologe Expression des *Prunus amygdalus HNL5* Gens in Bakterien und Eukaryonten.

Ziel dieses Experiments war die Konstruktion eines Plasmides, aus dem ein für *Prunus amygdalus HNL5* kodierendes DNA Fragment für den Einbau in verschiedene Expressionsvektoren durch Ausschneiden mit Restriktionsendonukleasen gewonnen werden kann. Dabei wurden durch PCR Amplifikation über entsprechende Primer an den Enden des in pBSPamHNL5orf enthaltenen *Prunus amygdalus HNL5* Gens geeignete Sequenzen addiert.

Mit den Primern PCRHNL5-a und PCRHNL5-e wurde das Insert des Plasmids pBSPamHNL5orf mittels PCR amplifiziert (10 ng DNA des Plasmids pBSPamHNL5orf als Template, 400 ng Primer PCRHNL5-a, 200 ng Primer PCRHNL5-e). Die PCR Reaktion erfolgte in 50 µl Ansätzen in 1x PCR Puffer (Qiagen), enthaltend 5 µl des dNTP (je 2 mM) Mix und 1,2 U "Hotstar" *Taq* DNA Polymerase (Qiagen). Es wurde folgendes Programm gefahren: 15 min 95°C, 30 Zyklen 1 min 95°C, 30 sec. 68°C, 1,5 min 72 °C und zum Abschluss 5 min 72 °C zur Herstellung vollständiger Produkte.

Das erhaltene DNA Fragment wurde nach Schneiden mit der Restriktionsendonuklease *Eco*RI in den Vektor pBSSK(-) (Stratagene, USA) einkloniert und durch Sequenzierung verifiziert. Das resultierende Plasmid wurde pBSPamHNL5ex benannt.

### Oligonukleotid-Primer:

PCRHNL5-a 
5'-
TCGAATTCGAGCTCGGTACCCGGGGATCCTCTAGAAATAATTTTGTTTAACTTTAA
GAAGGAGATATACATATGGAGAAATCAACAATGTCAGTTATACTATTTGTGTTGCA
TC-3'
 PCRHNL5-e

5'-CGAATTCGCCCTTTCGCATGCTCACATGGACTCTTGAATATTATGAATAGCCTC-
3'

### BEISPIEL 6

### Konstruktion von Expressionskonstrukten zur heterologen Expression des HNL5 Gens in Pichia pastoris.

DNA des Plasmids pBSPamHNL5ex wurde mit *EcoR*I geschnitten und das *HNL*-Fragment vom Vektoranteil mittels präparativer Gelelektrophorese getrennt. Nach Elution der *HNL*-Fragment DNA mittels Qiaex II Kit (Qiagen) wurde dieses über die *Eco*Rl Schnittstellen in die Plasmide pHILD2 und pGAPZ (Invitrogen, San Diego, CA, USA) einkloniert. Die richtige Orientierung der Inserts zu den Promotoren wurde mit Hilfe von Kontrollschnitten überprüft. Jeweils ein Klon mit einem korrekt orientierten Insert wurde ausgewählt und konserviert. Die korrekten Übergänge vom Vektoranteil zum eingebauten *HNL*-Fragment wurden durch Sequenzierung verifiziert. Diese beiden entstandenen Expressionsplasmide für *Pichia pastoris* wurden als pHILDPamHNL5a (für induzierbare Expression) und pGAPPamHNL5a (für konstitutive Expression) bezeichnet.

### BEISPIEL 7

### Induzierbare Expression des Prunus amygdalus HNL5 Gens in Pichia pastoris

DNA des Plasmids pHILDPamHNL5a wurde mit der Restriktionsendonuklease Notl geschnitten und in *Pichia pastoris* GS115 (Invitrogen, San Diego, CA, USA) transformiert. Die Transformation erfolgte nach den Vorschriften des "Pichia Expression Kit" (Invitrogen, San Diego, CA, USA). 100 Histidin-prototrophe Klone wurden nach den Vorschriften des "Pichia Expression Kit" in Flüssigmedium (500 ml Schüttelkulturen) angezüchtet und 48 Stunden mit Methanol induziert. Die Zellen wurden abzentrifugiert und mit Aufschlusspuffer (50 mM Tris-HCL, pH 7,6) zu einer optischen Dichte (OD₆₀₀) von 50.0 suspendiert und nach der "Glaskugelmethode" (Ausubel et al., Current Protocolls in Molecular Biology, Green Publishing Associates and Wiley-Interscience, New York, 1999) in einem "Merckenschlager" Homogenisator (Fa. Braun, Melsungen, BRD) aufgeschlossen. Sowohl die Kulturüberstände als auch die Zelllysate wurden auf HNL-Enzym Aktivität mit einem Standard Aktivitätsassay (analog WO 97/03204) unter Verwendung von racemischem Mandelonitril als Substrat bestimmt. Zwei Klone, die bei diesen Schüttelkolbenexperimenten die besten Aktivitäten im Kulturüberstand zeigten (*Pichia pastoris* PamHNL5-a37 und *Pichia pastoris* PamHNL5-a4) wurden ausgewählt und konserviert. Eine Analyse des Methanolverwertungstyps ergab den Phänotyp Mut⁺ (gute Verwertung von Methanol) für *Pichia pastoris* PamHNL5-a37 und den Phänotyp Mut^{s} (langsame Verwertung von Methanol) für *Pichia pastoris* PamHNL5-a4.

### BEISPIEL 8

### Konstitutive Expression des Prunus amygdalus HNL5 Gens in Pichia pastoris

Das Plasmid pGAPPamHNL5a wurde in P. pastoris GS115 transformiert. Die Transformation erfolgte nach den Vorschriften des Pichia Expression Kit der Invitrogen Corp (San Diego, CA, USA). Die Selektion von Transformanten erfolgte auf YPD Vollmediumplatten mit 100 mg/l Zeocin. 100 Zeocin-resistente Klone wurden in je 500 ml YPD Vollmedium angezüchtet und 96 Stunden bei 30°C geschüttelt. Die Zellen wurden abzentrifugiert und die HNL Aktivität im Kulturüberstand bestimmt. Ein Klon der die beste Aktivität im Kulturüberstand zeigte wurde konserviert und mit *Pichia pastoris* PamHNL5-a2 bezeichnet.

### BEISPIEL 9

### Produktion von HNL mit einem mit dem HNL5 Gen aus Prunus amygdalus transformierten Stamm von Pichia pastoris (durch Methanol indizierbares Expressionssystem) im Laborbioreaktor.

Die Züchtung von *Pichia pastoris* PamHNL5-a37 erfolgte in einem Standard-Laborbioreaktor (42 L Gesamtvolumen) in einem dreiphasigen Verfahren. Dieses bestand aus einer ersten exponentiellen und einer zweiten linearen Wachstumsphase zur Bildung von Biomasse und einer anschließenden Expressionsphase zur Bildung des rekombinanten *Prunus amygdalus* HNL Enzyms, wobei im Prinzip nach der für die Produktion von rekombinanter *Hevea brasiliensis* HNL beschriebenen Methode (Hasslacher, M., Schall, M., Hayn, M., Bona, R., Rumbold, K., Lückl, J., Griengl, H., Kohlwein, S.D., Schwab, H.: High level intracellular expression of hydroxynitrile lyase from the tropical rubber tree Hevea brasiliensis in microbial hosts. Protein Expression and Purification 11, 61-71, 1997) vorgegangen wurde.

Im Detail wurden folgende Bedingungen eingehalten:

Die Chemikalien 1.-8., bemessen für 20 Liter wurden in 15 Liter Wasser gelöst im Bioreaktor vorgelegt und zusammen mit dem Reaktor bei 121°C für 1 Stunde sterilisiert. Nach Abkühlung auf 29°C wurde der pH Wert im Medium mit Ammoniak (Chemikalium 9, in steriler Zulaufflasche vorgelegt) auf pH 5,0 eingestellt. Danach wurden ca. 200 ml einer steril filtrierten Spurenelemente-Lösung (Chemikalien 10-18, entsprechende Mengen für 20 L) über eine Zulaufflasche in den Bioreaktor eingebracht.

Der so vorbereitete Bioreaktor wurde mit 2 Liter Vorkultur, die in Schüttelkolben bei 30°C nach den im Handbuch des "Pichia Expression Kit" (Invitrogen Corp., San Diego, CA, USA) angegebenen Bedingungen angezüchtet wurde, beimpft. Die Kultivierung wurde bei einer konstanten Temperatur von 29°C durchgeführt. Durch Regelung der Belüftung (zwischen 15 und max. 40 Lit Luft/min) und der Rührerdrehzahl (zwischen 250 bis 500 Upm) wurde der O₂-Partialdruck auf einem Wert von größer als 30% der Sättigungskonzentration gehalten. Nach 21 Stunden war die Biomasse auf einen Wert im Bereich von 22 g/L Zelltrockengewicht (ZTG) angewachsen. Ab diesem Zeitpunkt wurde steriles Glycerin in konstanten kleinen Portionen in Abständen von 15 min zudosiert, wobei pro Stunde 130 g Glycerin zugesetzt wurden. In dieser zweiten linearen Wachstumsphase konnte in einem Zeitraum von 42 Stunden eine Biomassekonzentration im Bereich von 70 g/L ZTG erreicht werden.

Danach wurde die dritte Phase mit der Induktion der Expression durch Zudosierung von Methanol eingeleitet. Dabei wurde der Methanolgehalt in der Kulturbrühe auf einen Wert von 0,8-1 Gewicht % eingestellt. Zu Induktionsbeginn und nach zwei Tagen Induktion wurde jeweils eine weitere Portion sterile Spurenelementelösung (Chemikalien 10-18, entsprechende Mengen für 20 L, gelöst in ca. 200 ml Wasser) zugegeben. Nach 110 Stunden Induktionsphase konnte eine Enzymmenge von 110 U/ml Kulturbrühe erhalten werden. Nach Abtrennen der Zellen durch beispielsweise Zentrifugation kann eine Rohenzympräparation erhalten werden, die direkt für biokatalytische Umsetzungen eingesetzt werden kann.

Folgende Chemikalien wurden zur Herstellung des Kulturmediums benutzt (Menge pro Liter):

| | | |
|---|---|---|
| 1. | 85% ortho-Phosphorsäure | 21 ml |
| 2. | CaSO₄ | 0,9 g |
| 3. | K₂SO₄ | 14,3 g |
| 4. | MgSO₄.7H₂O | 12,2 g |
| 5. | KOH (Chemikalien 1 bis 5 in p.a. Qualität) | 3,3 g |
| 6. | Glycerin, technische Qualität. | 50 ml |
| 7. | Entionisiertes Wasser, Hausqualität, Leitfähigkeit | 5,5-9,1 µS/cm |
| 8. | Antischaummittel 10 % Acepol 83E Carl Becker Chemie GmbH, Hamburg, Deutschland | 1ml |
| 9. | 25%Ammoniak, technische Qualität | |

Spurenelemente und Vitamin H (alle Chemikalien in p.a. Qualität) :

| | | |
|---|---|---|
| 10. | Biotin | 0,8 mg |
| 11. | CuSO₄.5H₂O. | 24,0 mg |
| 12. | Kl | 0,32 mg |
| 13. | MnSO₄.H₂O. | 12,0 mg |
| 14. | Na₂MoO₄.2 H_{2O} | 0,2 mg |
| 15. | H₃BO₃ | 0,08 mg |
| 16. | CoCl₂ | 2,0 mg |
| 17. | ZnSO₄.7H₂O | 80 mg |
| 18. | Fe(II)SO₄.7H₂O | 260 mg |

### BEISPIEL 10:

### Konstruktion eines Klones zur Expression des Prunus amygdalus HNL5 Gens als Fusionsprotein mit N-terminalen und C-terminalen Anteilen der Glukoseoxidase von Aspergillus niger.

Diese Konstruktion wurde so konzipiert, dass das gebildete Fusionsprotein über die heterologe Signalsequenz in den sekretorischen Weg gelenkt wird.

Durch eine PCR in einem 50 µl Ansatz in 1x PCR Puffer (Qiagen) mit je 100 pmol der Primer Glucox2 und Glucoxct, 10 ng Plasmid pPamHNL5orf als Template , 5 µl dNTP (je 2 mM) Mix, und 1,2 U "Hotstar" *Taq* Polymerase (Qiagen) wurden das C-terminale und das N-terminale Ende des *HNL5* Gens durch eine von der Glukoseoxidase abgeleitete Sequenz ersetzt bzw. verkürzt. (Programm: 15 min 95 °C, 30 x: 1 min 95°C, 1 min 68 °C, 2 min 72°C, und zum Abschluss 10 min 72°C)

In einer 2. PCR in einem 50 µl Ansatz in 1x PCR Puffer(Qiagen) mit 0,1 µl Produkt aus der ersten PCR als Template, je 100 pmol der primer Glucox1 und Glucoxct, 2 µl dNTP (je 2 mM) Mix, und 2, 5 U Pwo Polymerase (Roche Diagnostics, Mannheim, Deutschland), (Programm: 5 min 95°C, 30 mal: 1 min 95 °C, 0,5 min 68 °C, 3 min 72 °C und zum Abschluss 3 min 72°C) wurde abschließend der 5' Bereich des Gens ergänzt.

Über die gleichzeitig eingeführten *Eco*RI Schnittstellen an den Enden des DNA Fragments wurde das PCR Produkt nach Schneiden mit *E*coRI in das Plasmid pHILD2 (Invitrogen, San Diego, CA, USA) eingebaut. Ein Klon mit der richtigen Orientierung des Inserts zum *aox* Promoter des pHILD2 Plasmids wurde durch Sequenzieren der Übergangsbereiche von Vektor zu Insert verifiziert und konserviert. Das auf diese Weise konstruierte Plasmid wurde mit pHILDPamHNL5gox bezeichnet.

Mit Notl linearisierte DNA des Plasmids pHILDPamHNL5gox wurde in den Stamm *Pichia pastoris* GS115 sowie in den proteasedefizienten Stamm *Pichia pastoris* SMD1168 transformiert. Von jedem Ansatz wurden mehrere Histidin-prototrophe Klone in Schüttelkolben kultiviert und die HNL-Aktivität im Kulturüberstand (Standard-assay) bestimmt. Diese Experimente wurden analog wie in Beispiel 7 beschrieben durchgeführt. Im Kulturüberstand war bei einigen Klonen HNL Aktivität aufzufinden womit festgestellt werden konnte, daß die Signalsequenz des *Aspergillus niger gox* Gens befähigt ist, das heterologe HNL5 Protein bei Expression in *Pichia pastoris* in den sekretorischen Weg zu dirigieren.

### VERWENDETE OLlGONUKLEOTID-PRIMER

GLUCOX1 
5'-
CACGAATTCATCATGCAGACTCTCCTTGTGAGCTCGCTTGTGGTCTCCCTCGCTG
CGGCCCTGCCACACTAC – 3'
 GLUCOX2 
5'-
TGCGGCCCTGCCACACTACATCAGGAGCAATGGCATTGAAGCCTACAACGCCACT
GATACAAGCTCGGAAGGATC-3'
 GLUCOXCT
5'-GAATTCGCATGCGGCCGCTCACTGCATTGACCTTTCTTGCAGGATTTGAAG-3'

Die Nukleinsäuresequenz des für ein sekretorisches Hybridprotein (PamHNL5xGOX) mit HNL-Aktivität codierenden DNA Fragments ist in Figur 4 dargestellt, die daraus abgeleitete Aminosäuresequenz ist in Figur 5 wiedergegeben. Ein Vergleich der Aminosäuresequenzen der HNL5 aus Prunus amygdalus und des Hybridproteins PamHNL5xGOX ist aus Figur 6 zu entnehmen.

### BEISPIEL 11:

Rekombinantes Protein mit HNL-Aktivität, das mit dem rekombinanten Stamm *Pichia pastoris* PamHNL5-a37 wie in Beispiel 9 beschrieben produziert wurde, wurde einer Analyse der Glykosylierung durch Verdau mit Endoglykosidasen unterzogen. Dazu wurden 100 ml des Kulturüberstandes durch Ultrafiltration (Biomax 30,000 NMWL, Millipore, Bedford, MA, USA) 10 fach konzentriert. Die Proben 1-2 wurden mit N-Glycosidase F (N-Glykosidase F Kit, Roche Diagnostics, Mannheim, D) behandelt.

Bei den Proben 4-5 wurde eine HNL Präparation aus Mandeln der Fa. Roche verwendet und bei den Proben 6 und 7 wurde der aufkonzentrierte Kulturüberstand mit Endoglykosidase H (Roche, Diagnostics, Mannheim, D) behandelt. Alle Ansätze wurden in 10 µl Gesamtvolumen durchgeführt.

| | |
|---|---|
| Std. | Molekulargewichtsstandard aus dem N-Glykosidase F Kit (5 µl =5 µg) |
| Probe 1: | 2 U *Pam*HNL5 mit 2,4 U Enzym nach Vorschrift des Kits behandelt. |
| Probe 2: | 2 U *Pam*HNL5 mit 2,4 U Enzym nach Vorschrift des Kits jedoch ohne Denaturierungspuffer und ohne Hitzedenaturierung behandelt |
| Probe 3: | 2 *Pam*HNL5 ohne Behandlung |
| Probe 4: | 0,25 U der Roche R-HNL Präparation grade III aus Mandeln (10,3 U/mg) behandelt mit 2,4 U N-Glycosidase F nach Vorschrift des Kits |
| Probe 5: | 0,25 U der Roche Präparation grade III (10,3 U/mg) unbehandelt |
| Probe 6 | 2,4 U *Pam*HNL5 wurden mit 50 mU Endoglykosidase H in 20 mM Phosphatpuffer, ohne Denaturierung für 12 Stunden bei 37°C inkubiert. |
| Probe 7 | 2,4 U *Pam*HNL5 wurden mit 5 mU Endoglykosidase H in 20 mM Phosphatpuffer, 0,2% SDS, 0,4 % Merkaptoethanol für 12 Stunden bei 37°C inkubiert. |

Nach den Behandlungen mit den Glykosidasen wurden die Proben auf einem 12-prozentigen SDS-Polyacrylamid Gel getrennt und mit Commassie Blue gefärbt.

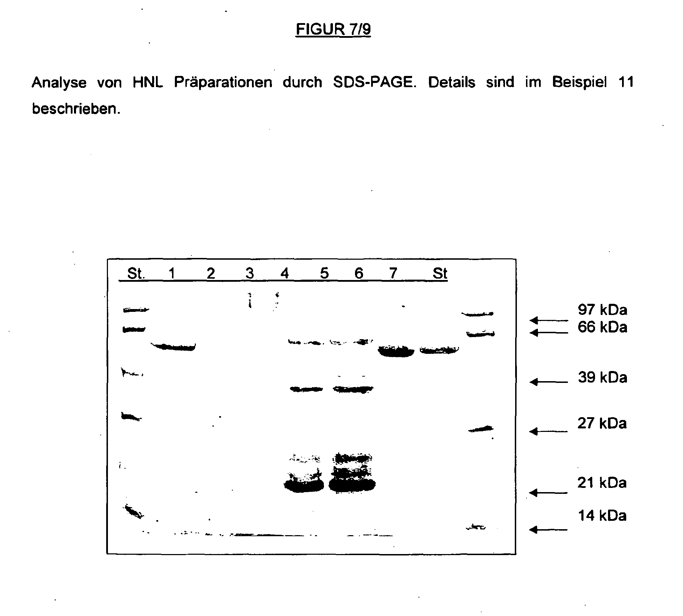

Diese Ergebnisse (siehe Figur 7) zeigen, dass ein Grossteil der an *Pam*HNL5 gebundenen Oligosaccharide durch Endoglykosidase H auch ohne Denaturierung des *Pam*HNL5 Proteins entfernt werden kann.
Durch die Abspaltung der Oligosaccharide entsteht aus einem bei Größen von 70 bis über 100 kDa sichbaren Proteinschmier eine scharfe Bande bei etwa 60 kDa, was dem berechneten Molekulargewicht eines nicht glykosylierten *Pam*HNL5 Proteins entspricht.

Eine vergleichbare Proteinbande ist im Roche Präparat nicht oder nur in stark untergeordnetem Ausmass vorhanden. Außerdem kann kein signifikanter Unterschied zwischen einer unbehandelten Proteinpräparation und einer mit Endoglykosidase F behandelten Präparation gesehen werden. Aus diesem Befund kann eindeutig festgestellt werden, dass sich das rekombinante *Pam*HNL5 Enzym komplett vom aus Mandelkernnen gewonnenen Enzymmaterial unterscheidet.

### BEISPIEL 12:

### Klonierung eines genomischen DNA Fragmentes mit der kodierenden Region des HNL1 Gens von Prunus amygdalus.

Ein genomisches DNA Fragment mit der kodierenden Region des *HNL1* Gens von *Prunus amygdalus* wurde mit den Primern mandlp2f (5'-ACTACGAATTCGACCATGGAGAAATCAAC-3') und ecpamHNL1e (5'-CAGAATTCGCCCTTGTGCATGCATCGATTAAAGAACCAAGGATGCTGCTGAC-3') aus genomischer Mandel DNA (Präparation siehe Beispiel 1) mit Hilfe einer PCR amplifiziert.

Die Amplifikation wurde in 50µl Reaktionen mit 1,2 units "Hotstar" DNA Polymerase (Qiagen GmbH, Hilden, Germany), je 10 pmol der beiden primer, 2µl eines dNTP Mix (je 5 mM) und 100 ng genomischer Mandel DNA in Standard PCR Puffer (Qiagen GmbH, Hilden, Germany),) durchgeführt. Folgendes PCR Programm wurde verwendet:
15 min 95°C, dann 10 Zyklen mit 1 min bei 94°C, 1 min 45°C und 1 min 20 sec bei 72°C, gefolgt von 30 Zyklen mit 1 min bei 94°C, 1 min bei 64 °C und 1 min 20 sec bei 72°C sowie einem abschließenden "extension" Schritt für 5 min bei 72°C.

Eine Analyse der erhaltenen DNA ergab, dass durch diese PCR mehrere DNA Fragmente unterschiedlicher Größe erhalten wurden. Amplifizierte DNA wurde in einem präparativen Agarosegel getrennt. DNA aus einer Bande in der für das *HNL1* Gen zu erwartenden Grösse von ca 2,1 kb wurde aus diesem Agarosegel isoliert (Qiaquick Gel Extraction Kit, Qiagen GmbH, Hilden, Germany),). Die erhaltene DNA wurde nach Verdau mit der Restriktionsendonuklease *Eco*RI über die *Eco*RI Schnittstelle in den Kloniervektor pBSSK(-)(Stratagene Cloning Systems, La Jolla, CA, USA) einkloniert. 5 Klone mit entsprechenden Inserts wurden isoliert und die inserts mittels der "primer walking" Strategie sequenziert. Ein Klon, der der so erhaltenen Konsensussequenz entsprach wurde ausgewählt und das enthaltene Plasmid mit pSKpamHNL1_5_3 bezeichnet.

Zur Verifizierung und endgültigen Bestimmung der DNA Sequenz des *HNL1* Gens von *Prunus amgdalus* wurde ein weiteres genomisches DNA Fragment mit den Primern mandlp3f (5'-ACTACGAATTCGACCATGGAGAAATCAACAATG-3') und pamHNL1end (5'-ATGCTGCTGACTTGAGGGAATC-3') amplifiziert. Die Amplifikation wurde in 50µl Reaktionen mit 2,5 units "Hotstar" DNA Polymerase (Qiagen GmbH, Hilden, Germany), je 10 pmol der beiden Primer, 2 µl eines dNTP Mix (je 5 mM) und 50 ng genomischer Mandel DNA in Standard PCR Puffer (Qiagen GmbH, Hilden, Germany) durchgeführt. Folgendes PCR Programm wurde verwendet:
15 min 95°C, dann 5 Zyklen mit 1 min bei 94°C, 30 sec bei 55°C und 2 min bei 72°C, dann 30 Zyklen mit 1 min bei 94°C, 30 sec bei 68 °C und 2 min bei 72°C sowie einem abschliessenden "extension" Schritt für 7 min bei 72°C.

Nach Auftrennung des PCR Produktes in einem Agarosegel wurde eine einzelne DNA Bande detektiert. Das PCR Produkt wurde mittels Qiaquick Gel Extraction Kit (Qiagen GmbH, Hilden, Germany) aufgereinigt und mittels "Primer walking" Strategie direkt sequenziert.

Die DNA Sequenz ist in Abbildung 8 dargestellt. Mögliche Introns wurden durch ihre allgemeinen 5' und 3' splice sites sowie ihre Homologie zum *Prunus serotina HNL1* Gen identifiziert. Die Nukleotide in den Bereichen der drei Introns sind zur Erkennung der Intronbereiche in Kleinbuchstaben gezeigt. Das klonierte insert im Plasmid pSKpamHNL1_5_3 weist die gleiche Sequenz auf.

Die Proteinsequenz des HNL1 Proteins von *Prunus amygdalus* wurde aus dem kodierenden Bereich der DNA Sequenz abgeleitet und ist in der Abb. 9 dargestellt.

## Patentansprüche

1. Isolierte Nukleinsäure, welche die in Figur 8 abgebildete Nukleotidsequenz von Nukleotid 1 bis Nukleotid 2083 durchgehend oder diese Sequenz abzüglich der Intron-Bereiche von den Nukleotiden 104 bis 249, 907 bis 1047 und 1889 bis 1993 umfasst.

2. Isolierte Nukleinsäure nach Anspruch 1, welche die in Figur 8 abgebildete Nukleotidsequenz umfasst.

3. Rekombinante Proteine, umfassend eine Aminosäuresequenz gemäß Figur 9.

4. Rekombinante Proteine nach Anspruch 3, **dadurch gekennzeichnet, dass** sie eine wirtsspezifische Glykosylierung aufweisen.

5. Rekombinante Proteine nach Anspruch 3, **dadurch gekennzeichnet, dass** sie durch Expression in einem eukaryontischen Mikroorganismus hergestellt werden.

6. Rekombinante Proteine nach Anspruch 3, **dadurch gekennzeichnet, dass** sie durch Expression in einem Pilz hergestellt werden.

7. Rekombinante Proteine nach Anspruch 3, **dadurch gekennzeichnet, dass** das Protein die aus der Nukleotidsequenz der isolierten Nukleinsäure nach Anspruch 1 oder 2 abgeleitete Aminosäuresequenz aufweist.

8. Verwendung von Proteinen nach einem der Ansprüche 3 - 7 zur Herstellung von (S)- oder (R)-Cyanhydrinen.

9. Verfahren zur Herstellung von (S)- oder (R)-Cyanhydrinen, **dadurch gekennzeichnet, dass** aliphatische, aromatische oder heteroaromatische Aldehyde und Ketone in Anwesenheit eines Cyanidgruppendonors mit Proteinen nach einem der Ansprüche 3-7 im organischen, wässrigen oder 2-Phasensystem oder in Emulsion umgesetzt werden.

## Claims

1. Isolated nucleic acid which comprises the nucleotide sequence shown in Figure 8 from nucleotide 1 right through to nucleotide 2083 or this sequence minus the intron regions of nucleotides 104 to 249, 907 to 1047 and 1889 to 1993.

2. Isolated nucleic acid according to Claim 1, which comprises the nucleotide sequence shown in Figure 8.

3. Recombinant proteins comprising an amino acid sequence as set forth in Figure 9.

4. Recombinant proteins according to Claim 3, **characterized in that** they comprise host-specific glycosylation.

5. Recombinant proteins according to Claim 3, **characterized in that** they are prepared by expression in a eukaryotic microorganism.

6. Recombinant proteins according to Claim 3, **characterized in that** they are prepared by expression in a fungus.

7. Recombinant proteins according to Claim 3, **characterized in that** the protein has the amino acid sequence derived from the nucleotide sequence of the isolated nucleic acid according to Claim 1 or 2.

8. Use of proteins according to any of Claims 3-7 for preparing (S)- or (R)-cyanohydrins.

9. Process for preparing (S)- or (R)-cyanohydrins, **characterized in that** aliphatic, aromatic or heteroaromatic aldehydes and ketones are reacted with proteins according to any of Claims 3-7 in an organic, aqueous or 2-phase system or in emulsion in the presence of a cyanide group donor.

## Revendications

1. Acide nucléique isolé, qui comprend la séquence nucléotidique allant du nucléotide 1 au nucléotide 2083, représentée sur la figure 8, ou cette séquence moins les régions d'introns des nucléotides 104 à 249, 907 à 1047 et 1889 à 1993.

2. Acide nucléique isolé, selon la revendication 1, qui comprend la séquence nucléotidique représentée sur la figure 8.

3. Protéines recombinantes, comprenant une séquence d'aminoacides selon la figure 9.

4. Protéines recombinantes selon la revendication 3, **caractérisées en ce qu'**elles présentent une glycosylation spécifique de l'hôte.

5. Protéines recombinantes selon la revendication 3, **caractérisées en ce qu'**elles sont produites par expression dans un micro-organisme eucaryote.

6. Protéines recombinantes selon la revendication 3, **caractérisées en ce qu'**elles sont produites par expression dans un champignon.

7. Protéines recombinantes selon la revendication 3, **caractérisées en ce que** la protéine présente la séquence d'aminoacides dérivée de la séquence nucléotidique de l'acide nucléique isolé, selon la revendication 1 ou 2.

8. Utilisation de protéines selon l'une quelconque des revendications 3 à 7, pour la préparation de (S)- ou (R)-cyanohydrines.

9. Procédé pour la production de préparation de (S)- ou (R)-cyanohydrines, **caractérisé en ce qu'**on fait réagir des cétones et aldéhydes aliphatiques, aromatiques ou hétéroaromatiques en présence d'un donneur de groupes cyanure avec des protéines selon l'une quelconque des revendications 3 à 7, dans le système organique, aqueux ou en 2 phases ou en émulsion.
